# EUROPEAN PATENT APPLICATION

(11) **EP 4 825 430 A1**
(43) Date of publication of application: **21.10.2026**
(21) Application number: 25171416.8
(22) Date of filing: 17.04.2025
(51) Int. Cl.: A61M 35/00

(54) **CONTAINER COMPRISING A FORMULATION OF DICLOFENAC**

(71) Applicant: Haleon CH SARL, 1197 Prangins (CH)
(72) Inventor: The designation of the inventor has not yet been filed
(74) Representative: Haleon Patent Department

(57) **Abstract**

The present invention relates to a container (100) comprising a topical formulation of diclofenac or pharmaceutically acceptable salt thereof for use in a method of increasing the efficiency of topical diclofenac administration. Particularly the container comprises: (i) an interior space containing the topical formulation of diclofenac or pharmaceutically acceptable salt thereof; (ii) an application surface (40) and an output channel (50), the output channel connecting the interior space with the application surface for dispensing the topical formulation onto the application surface; and (iii) a cap (1) configured to cover the application surface and close the output channel of the container.

## Description

### Technical Field

The present disclosure relates to containers comprising a topical formulation of diclofenac, or pharmaceutically acceptable salt thereof, for use in a method of increasing the efficiency of topical diclofenac administration. Particularly, the containers comprise an applicator surface for applying the topical formulation.

### Technical Background

There is an increasing regulatory and public concern associated with Pharmaceuticals in Environment (PIE) and legislative organizations/ regulatory agencies continue to establish limits to drive minimalization of the environmental exposure to medicines.

Currently the EU has the most developed requirements for Environmental Risk Assessment (ERA), which requires according to Regulation (EC) No 726/2004, Directive 2001/83/EC (produced following European Medicines Agency (EMA) guidance (EMEA/CHMP/ SWP/4447/00)).

Topical analgesics can enter the environment via greywater, for example household wastewater from sinks, released when a consumer washes their hands to remove residues following hand application of the topical analgesic. Another route of entry is from the unabsorbed product residue remaining on the skin after application. This residue may also be absorbed by clothing, entering the wastewater during laundry.

Product residue remaining on the skin after the application is significantly influenced by the concentration of topical analgesic in each dose and method of application.

Thus, there remains a need for application methods minimise environmental release of topical analgesics whilst maximising the efficacy of such topical products.

### Summary of the Invention

In a First Aspect there is provided a container comprising a topical formulation of diclofenac or pharmaceutically acceptable salt thereof for use in a method of increasing the efficiency of topical diclofenac administration.

Particularly the container comprises:
(i) an interior space containing the topical formulation of diclofenac or pharmaceutically acceptable salt thereof;
(ii) an application surface and an output channel, the output channel connecting the interior space with the application surface for dispensing the topical formulation onto the application surface; and
(iii) a cap configured to cover the application surface and close the output channel of the container; and

Particularly the method comprises:
(a) removing the cap to open the output channel;
(b) dispensing an effective amount of the topical formulation from the container onto the application surface;
(c) applying the topical formulation to the skin of a target area of the body using the application surface; and
(d) rubbing the topical formulation into the target area of the body using the application surface for at least 60 seconds.

Preferably, the topical formulation comprises from 0.5% to 5% w/w of diclofenac or equivalent amount of pharmaceutically acceptable salt thereof. In some embodiments the topical composition comprises diclofenac sodium. In other embodiments, pharmaceutically acceptable salts of diclofenac with organic bases of ammonia are used. For example, the ammonium salt, alkylamine salt dimethylamine, diethylamine, trimethylamine and a cyclic amine salt such as epolamine (hydroxyethylpyrrolidine salt) can be used. Preferably, the topical compositions comprise diclofenac sodium, diclofenac diethylammonium or diclofenac diethylamine. In some embodiments, the topical compositions comprise diclofenac sodium or diclofenac diethylammonium, particularly 1% w/w diclofenac sodium, 1.16% w/w diclofenac diethylammonium or 2.32% w/w diclofenac diethylammonium. In other embodiments, the topical compositions comprise diclofenac sodium or diclofenac diethylammonium, particularly 0.5% w/w to 0.9% w/w diclofenac sodium or 0.5% w/w to 1% w/w diclofenac diethylammonium.

Particularly the application surface comprises a massage portion comprising protrusions and/or recessions that serve to rub the topical formulation into a target area of the body brought into contact with the massage portion. In some embodiments the massage portion at least partially, optionally fully, surrounds the output channel so that topical formulation output from the output channel spreads over the massage portion.

Preferably the application surface, optionally the massage portion, comprises a dosage marking portion, such as a dosing ring marking, optionally a circular shaped dosing marking portion, optionally the dosing marking portion being arranged concentrically around the output channel.

Particularly the dosage marking portion surrounds the output channel at a predetermined distance, wherein spreading of the topical formulation on the application surface from the output channel to the dosage marking portion indicates a dosage of topical formulation.

Particularly the massage portion comprises an inner massage portion that is surrounded by the dosing marking portion and an outer massage portion that surrounds the dosing marking portion, wherein the inner massage portion surrounds the output channel.

In some embodiments, the massage portion comprises at least one hexagonal protrusion, the massage portion optionally comprising a honey-comb arrangement of hexagonal protrusions.

Particularly, the container may be flexible and a side wall may be squeezed to dispense the topical formulation from the interior space of the container. The container may in particular be a cream tube or a gel tube, such as an emulgel tube. In certain embodiments, the container is a flexible container that may be filled and sealed at the base by crimping. Preferably, the container is a squeeze tube.

Preferably the container comprises at least one material selected from the group consisting of silicon, LDPE, TPE, PE, PP, rubber, and PET.

In a Second Aspect, there is provided a kit comprising the container for use of the First Aspect and instructions for use.

Particularly, the kit comprises instructions to dispense an effective amount of the topical formulation from the container onto the application surface, apply the topical formulation to the skin of a target area of the body using the application surface, and rub the topical formulation into the target area of the body using the application surface for at least 60 seconds.

More particularly the kit comprises instructions to rub the topical formulation into the target area of the body using the application surface for at least 60 seconds until fully absorbed.

### Brief Description of the Drawings

- **Fig. 1**: shows an exemplary container (100) for use in the method, in this case a flexible tube with a cap (1) covering an application surface.
- **Fig. 2**: shows the container (100) with the cap removed and the applicator surface (40) visible.
- **Fig. 3**: provides two close-up views of an exemplary applicator surface.

### Detailed Description

After application of a topical product by hand, up to 90% of the total dose applied may be wasted as a result of absorption by the skin of the hand and/or residue remaining on the hand that is removed by hand-washing.

The present Inventors have discovered a method for application of topical compositions of diclofenac or pharmaceutically acceptable salts thereof that minimise contact with a patient's hand whilst maximising product absorption by the skin of a target area of the body. The method utilizes an applicator that minimizes hand contact and reduces the amount of residue remaining on the surface of the skin at the target site after application. Surprisingly, the method reduces loss of diclofenac such that the similar clinical benefits can be achieved using a lower dose of diclofenac, for example 0.5g per dose vs 2g to 4g per dose currently used, or lower concentration of diclofenac, for example 0.5% w/w vs 1% w/w or 1.16% w/w.

Particularly the container used comprises:
(i) an interior space containing the topical formulation of diclofenac or pharmaceutically acceptable salt thereof;
(ii) an application surface and an output channel, the output channel connecting the interior space with the application surface for dispensing the topical formulation onto the application surface; and
(iii) a cap configured to cover the application surface and close the output channel of the container.

When the topical formulation is dispensed from the output channel of the container, it reaches the application surface and may start spreading on the application surface. Optionally, the geometry of the application surface may be chosen symmetrically around an output channel opening, so that the topical formulation spreads out radially from the output channel opening. In other words, the topical formulation may start forming a more or less round dot of topical formulation which starts growing radially around the output channel opening when more topical formulation is output.

The application surface may comprise a massage portion. The massage portion may be a smooth surface or may comprise protrusions and/or recessions that serve to rub the topical formulation into a target zone brought into contact with the massage portion while moving the cap base body. The target zone may, e.g., be a piece of skin with which the user makes contact to apply the topical formulation via the application surface.

Dispensing of the topical formulation may be achieved by exerting pressure from the inside. For example, a user may apply pressure to the container, so that the topical formulation is squeezed out of the output channel and onto the application surface.

The massage portion may, e.g., extend circularly around an output channel opening and/or comprise several output channel openings.

The massage portion may comprise protrusions that extend beyond a base surface of the application surface. Alternatively, the massage portion may comprise recessions that appear as indentations with respect to a base surface of the application surface. According to some embodiments, the massage portions may comprise at least one protrusion and at least one recession. According to other embodiments, the massage portion may be a substantially smooth surface having no grooves, protrusions, or the like, having a surface roughness (Ra) of less than or equal to 0.25 microns, for example measured using a profilometer. According to some embodiments, the massage portion may comprise an undulating surface.

The method of use comprises:
(a) removing the cap to open the output channel;
(b) dispensing an effective amount of the topical formulation from the container onto the application surface;
(c) applying the topical formulation to the skin of a target area of the body using the application surface; and
(d) rubbing the topical formulation into the target area of the body using the application surface for at least 60 seconds, preferably until fully absorbed.

The use of an applicator enables the topical formulation to be precisely applied to the anatomical structure where pain is being experienced. For example, when applying the topical formulation to the knee, preferably the formulation is applied to the patella (kneecap) rather than to the surrounding tissue.

The term "topical formulation" is to be construed broadly in this context and may refer to a formulation including an active ingredient, such as a medication, a drug, or a botanical, as well as a so-called vehicle. The vehicle may comprise or consist of water, oil, alcohol, propylene glycol mixed with preservatives, emulsifiers, absorption promoters, and fragrances, etc. The term topical formulation thus covers any fluid provided with an active ingredient, wherein the fluid is to be dispensed from a container for use. Particularly, the topical formulation has a viscosity greater than that of water (1 cPs), for example a viscosity of at least 100 centipoise (cPs), for example within the range of 100 cPs to 80,000 cPs, such as at least 500 cPs, for example within the range of from 500 cPs to about 80,000 cPs. In certain embodiments the topical formulation has a viscosity of at least 1000 cPs, for example from 1000 cPs to 80,000 cPs. Particular ranges include from 500 cPs to 25,000 cPs, 5000 cPs to 10,000 cPs, 10,000 cPs to 15,000 cPs or 15,000 cPs to 20,000 cPs. Particularly viscosity values may be measured using methods known in the art, for example, using a Brookfield viscometer at a temperature of 25° C.

The topical formulation may be provided in various forms and selected from group comprising gel, emulgel, lotion, cream, oil, emulsion and ointment. Particularly, the topical formulation is a cream, gel or emulgel having a viscosity of at least 100 centipoise. More particularly, the topical formulation is a cream, gel or emulgel having a viscosity of at least 100 centipoise measured using a Brookfield viscometer at a temperature of 25° C. In an embodiment, the topical formulation is a cream, gel or emulgel containing diclofenac sodium, particularly a cream, gel or emulgel containing diclofenac sodium having a viscosity of at least 100 centipoise. In specific embodiments, the topical formulation is a combination of diclofenac sodium, ammonia, carbomer homopolymer type c, cococaprylate/caprate, isopropyl alcohol, mineral oil, polyoxyl 20 cetostearyl ether, propylene glycol, and water.

The following is a description of an exemplary container and applicator for use in the method.

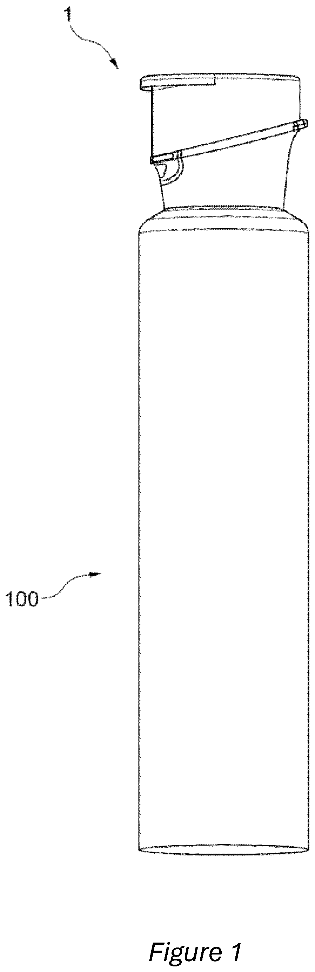

Figure 1 shows a container (100), in this case a flexible tube with a cap (1) covering an application surface.

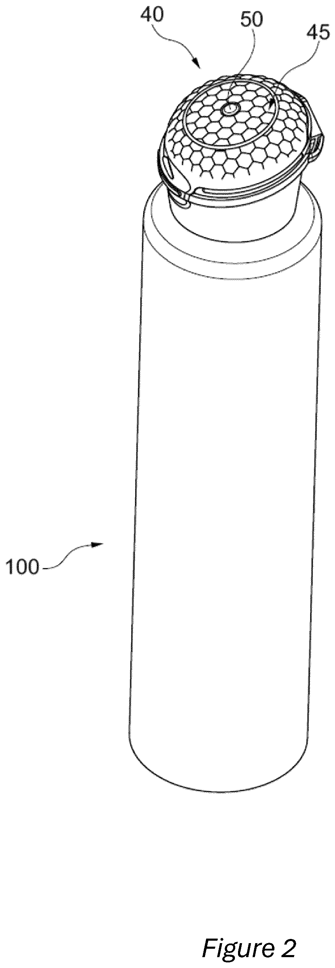

Figure 2 shows the container (100) with the cap removed. The application surface (40) comprises a massage portion (45) comprising protrusions that serve to rub the topical formulation into a target zone (e.g., a skin surface) brought into contact with the massage portion (45).

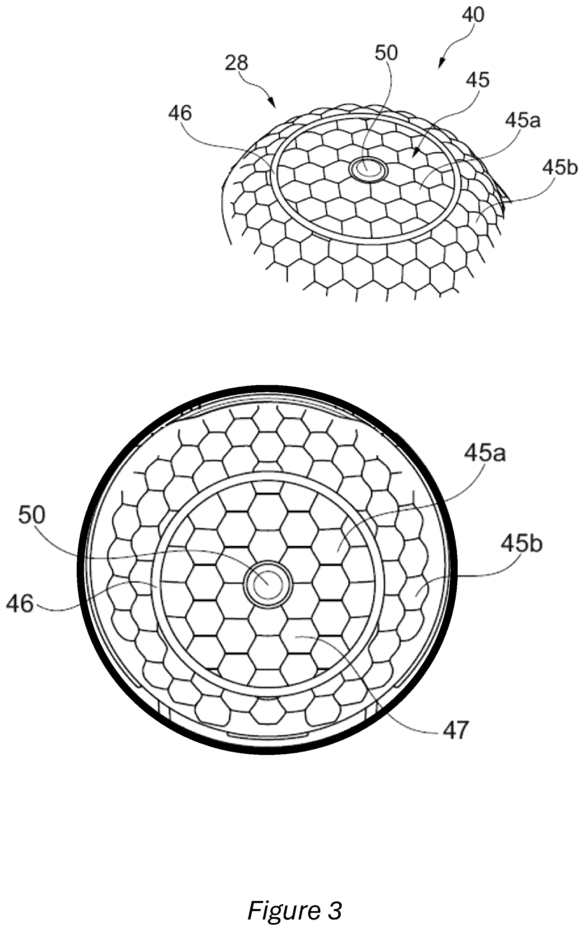

Figure 3 provides two close-up views of an exemplary application surface in greater detail. The massage portion (45) comprises a dosage marking portion (46), in this case with a ring-shape, being arranged concentrically around the output channel (50). The dosage marking portion (46) surrounds the output channel (50) at a predetermined distance and indicates a predetermined amount of a topical formulation by virtue of being reached by a topical formulation spreading on the application surface (40).

The massage portion (45) comprises an inner massage portion (45a) that is surrounded by the dosing marking portion (46) and an outer massage portion (45b) that surrounds the dosing marking portion (46). The inner massage portion (45a) and the outer massage portion (45b) both concentrically surround the output channel (50). The massage portion (45) fully surrounds the output channel (50) so that topical formulation output from the output channel (50) homogeneously spreads over the massage portion (45) (at least if the container and the cap are held more or less upright).

The massage portion (45) comprises hexagonal protrusions (47). The hexagonal protrusions (45) are together arranged to form a honey-comb arrangement.

More generally, the applicator surface (40) comprises a central flat portion and an outer rounded circumference portion surrounding the central flat portion, wherein the hexagonal protrusions (47) protrude from the central flat portion, and there are some further protrusions (47) at the rounded circumference portion. The outer part of the rounded circumference portion is smooth and comprises no protrusions or recessions.

### Examples

A clinical trial was designed to quantify the amount of diclofenac in rinse water (greywater) after application of a test product (Voltaren^{®} Schmerzgel forte; 2.32%) containing 2.32% w/w diclofenac diethylammonium either by hand or using an applicator and subsequent washing and/or wiping.

The trial is an open label, crossover study. Participants with healthy skin on the elbows and hands are enrolled. The amount of diclofenac in rinse water after application of the test product (Voltaren^{®} Schmerzgel forte; 2.32%) containing 2.32% diclofenac diethylammonium is assessed after three different washing methods, with and without use of an applicator in a crossover design. There is a wash-out period of at least 2 days in between each method. Approximately 40 healthy male and female participants between the ages of 18 and 70 years old are enrolled in this study.

### Arms and Interventions

Experimental: Voltaren^{®} Schmerzgel forte; 2.32% Applied Without Applicator (Method 1). Participants will apply the test product (Voltaren^{®} Schmerzgel forte; 2.32%) topically on the assigned test area (left or right elbow, depending on the handedness of the participant) using 3 different methods, once on Days 1, 3 and 5 as per crossover assignment. There will be a washout period of at least 2 days between each method. Method 1: Participants will apply the test product on the assigned test area with one hand by gently massaging in for 60 seconds and will wash their hands and 5 minutes after application, will wash the test area according to the washing procedure on Days 1, 3 and 5 as per crossover assignment. The water of each washing step will be collected separately.

Drug: Voltaren^{®} Schmerzgel forte; 2.32% (Test Product) Voltaren^{®} Schmerzgel forte; 2.32% containing 2.32% diclofenac diethylammonium.

Experimental: Voltaren^{®} Schmerzgel forte; 2.32% Applied With Applicator (Method 2). Participants will apply the test product (Voltaren^{®} Schmerzgel forte; 2.32%) topically on the assigned test area (left or right elbow, depending on the handedness of the participant) using 3 different methods, once on Days 1, 3 and 5 as per crossover assignment. There will be a washout period of at least 2 days between each method. Method 2: Participants will apply the test product on the assigned test area with the applicator, followed by washing the test area 5 minutes after application (1st washing) and 5 minutes after 1st washing (2nd washing) on Days 1, 3 and 5 as per crossover assignment. The water of each washing step will be collected separately.

Drug: Voltaren^{®} Schmerzgel forte; 2.32% (Test Product) Voltaren^{®} Schmerzgel forte; 2.32% containing 2.32% diclofenac diethylammonium.

Experimental: Voltaren^{®} Schmerzgel forte; 2.32% Applied With Applicator (Method 3). Participants will apply the test product (Voltaren^{®} Schmerzgel forte; 2.32%) topically on the assigned test area (left or right elbow, depending on the handedness of the participant) using 3 different methods, once on Days 1, 3 and 5 as per crossover assignment. There will be a washout period of at least 2 days between each method. Method 3: Participants will apply the test product on the assigned test area with the applicator followed by wiping the test area with a paper towel and washing the test area and hands according to the washing procedure after 5 minutes of application on Days 1, 3 and 5 as per crossover assignment. The water of the washing step will be collected.

Drug: Voltaren^{®} Schmerzgel forte; 2.32% (Test Product) Voltaren^{®} Schmerzgel forte; 2.32% containing 2.32% Diclofenac-N-Ethylethanamin.

### Primary Outcome Measure

Concentration of Voltaren^{®} Schmerzgel forte; 2.32% (Diclofenac) in Water (Milligrams per Liter [mg/L]).

The concentration of diclofenac in rinse water samples (mg/L) collected after application of the test product (Voltaren^{®} Schmerzgel forte; 2.32%) either by hand or using an applicator and subsequent washing will be determined with High-performance liquid chromatography (HPLC). [Time Frame: Day 1, Day 3 and Day 5].

Amount of Test Product Residues by Gravimetrical Measurements (Milligrams [mg]). Test product (Voltaren^{®} Schmerzgel forte; 2.32%) residues will be collected using Method 3 (application of the test product using an applicator and subsequent wiping with a paper towel and washing). The paper towels will be weighed before and after application. The amount of test product residues (mg) in paper towel will be determined using gravimetrical measurements. [Time Frame: Day 1, Day 3 and Day 5].

It will be apparent to those skilled in the art that various modifications and variations can be made in the disclosed devices and systems without departing from the scope of the disclosure. Other aspects of the disclosure will be apparent to those skilled in the art from consideration of the specification and practice of the features disclosed herein. It is intended that the specification and examples be considered as exemplary only. Many additional variations and modifications are possible and are understood to fall within the framework of the disclosure.

## Claims

1. A container comprising a topical formulation of diclofenac or pharmaceutically acceptable salt thereof for use in a method of increasing the efficiency of topical diclofenac administration, wherein the container comprises:
(i) an interior space containing the topical formulation of diclofenac or pharmaceutically acceptable salt thereof;
(ii) an application surface and an output channel, the output channel connecting the interior space with the application surface for dispensing the topical formulation onto the application surface; and
(iii) a cap configured to cover the application surface and close the output channel of the container; and
wherein the method comprises:
(a) removing the cap to open the output channel;
(b) dispensing an effective amount of the topical formulation from the container onto the application surface;
(c) applying the topical formulation to the skin of a target area of the body using the application surface; and
(d) rubbing the topical formulation into the target area of the body using the application surface for at least 60 seconds.

2. The container for use of claim 1, wherein the topical formulation comprises from 0.5% to 5% w/w of diclofenac or equivalent amount of pharmaceutically acceptable salt thereof.

3. The container for use of claim 2, wherein the application surface comprises a massage portion comprising protrusions and/or recessions that serve to rub the topical formulation into a target area of the body brought into contact with the massage portion.

4. The container for use of claim 3, wherein the massage portion at least partially, optionally fully, surrounds the output channel so that topical formulation output from the output channel spreads over the massage portion.

5. The container for use of any preceding claim wherein the application surface, optionally the massage portion, comprises a dosage marking portion, such as a dosing ring marking, optionally a circular shaped dosing marking portion, optionally the dosing marking portion being arranged concentrically around the output channel.

6. The container for use of claim 5, wherein the dosage marking portion surrounds the output channel at a predetermined distance, wherein spreading of the topical formulation on the application surface from the output channel to the dosage marking portion indicates a dosage of topical formulation.

7. The container for use of claim 6, wherein the massage portion comprises an inner massage portion that is surrounded by the dosing marking portion and an outer massage portion that surrounds the dosing marking portion, wherein the inner massage portion surrounds the output channel.

8. The container for use of any one of claims 3 to 7, wherein the massage portion comprises at least one hexagonal protrusion, the massage portion optionally comprising a honey-comb arrangement of hexagonal protrusions.

9. The container for use of any preceding claim, wherein the container is a squeeze tube.

10. The container for use of claim 9, comprising at least one material selected from the group consisting of silicon, LDPE, TPE, PE, PP, rubber, and PET.

11. A kit comprising the container for use of any preceding claim and instructions for use.

12. The kit of claim 11, wherein the kit comprises instructions to dispense an effective amount of the topical formulation from the container onto the application surface, apply the topical formulation to the skin of a target area of the body using the application surface, and rub the topical formulation into the target area of the body using the application surface for at least 60 seconds.

13. The kit of claim 12, wherein the kit comprises instructions to rub the topical formulation into the target area of the body using the application surface for at least 60 seconds until fully absorbed.
